(19) Europäisches Patentamt
European Patent Office
Office européen des brevets

(11) **EP 3 662 965 B1**

(12) **EUROPEAN PATENT SPECIFICATION**

(45) Date of publication and mention
of the grant of the patent:
**01.05.2024 Bulletin 2024/18**

(21) Application number: **19200742.5**

(22) Date of filing: **29.12.2012**

(51) International Patent Classification (IPC):
**A61M 31/00** $^{(2006.01)}$

(52) Cooperative Patent Classification (CPC):
**A61B 5/4839; A61B 5/14532; A61B 5/7275;
G16H 20/17; G16H 40/63;** A61B 5/1451;
A61B 5/1495

(54) **METHOD AND APPARATUS FOR DETERMINING MEDICATION DOSE INFORMATION**

VERFAHREN UND VORRICHTUNG ZUR FESTLEGUNG VON MEDIKAMENTENDOSISINFORMATIONEN

PROCÉDÉ ET APPAREIL PERMETTANT DE DÉTERMINER DES INFORMATIONS DE DOSE DE MÉDICAMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.12.2011 US 201161582209 P**

(43) Date of publication of application:
**10.06.2020 Bulletin 2020/24**

(60) Divisional application:
**23191714.7 / 4 249 034**

(62) Document number(s) of the earlier application(s) in
accordance with Art. 76 EPC:
**12863276.7 / 2 797 660**

(73) Proprietor: **Abbott Diabetes Care Inc.
Alameda, CA 94502 (US)**

(72) Inventors:
• **TAUB, Marc Barry
Mountain View, CA 94040 (US)**
• **BUDIMAN, Erwin Satrya
Fremont, CA 94555 (US)**
• **KARAN, Jai
Walnut, CA 91789 (US)**
• **DUNN, Timothy Christian
San Francisco, CA 94117 (US)**

(74) Representative: **Booth, Catherine Louise
Mathys & Squire
The Shard
32 London Bridge Street
London SE1 9SG (GB)**

(56) References cited:
**US-A1- 2003 208 113    US-A1- 2005 119 540
US-A1- 2008 228 056    US-A1- 2009 143 725
US-A1- 2010 057 042    US-A1- 2010 121 170**

• **FRASER CAMERON ET AL: "Probabilistic
Evolving Meal Detection and Estimation of Meal
Total Glucose Appearance Author Affiliations",
JOURNAL OF DIABETES SCIENCE AND
TECHNOLOGY VOLUME DIABETES
TECHNOLOGY SOCIETY, vol. 3, no. 5, 1
September 2009 (2009-09-01), pages 1022-1030,
XP055311666, DOI:
10.1177/193229680900300505**

**Description**

BACKGROUND

The detection of the level of glucose or other analytes, such as lactate, oxygen or the like, in certain individuals is vitally important to their health. For example, the monitoring of glucose is particularly important to individuals with diabetes. Diabetics may need to monitor glucose levels to determine when insulin is needed to reduce glucose levels in their bodies or when additional glucose is needed to raise the level of glucose in their bodies.

Devices have been developed for continuous or automatic monitoring of analytes, such as glucose, in bodily fluid such as in the blood stream or in interstitial fluid. Some of these analyte measuring devices are configured so that at least a portion of the devices are positioned below a skin surface of a user, e.g., in a blood vessel or in the subcutaneous tissue of a user.

US 2009/143725 discloses a method and device for outputting one or more signals associated with a monitored analyte level of an individual, the one or more signals including a substantially real time monitored analyte level and a rate of change information associated with the monitored analyte level.

US 2010/057042 discloses a method and device for monitoring control parameters in a closed loop control operation including continuously monitoring glucose level and automatic administration of a medication. Closed loop operation parameters are monitored and a predictive modeling analysis of the monitored closed loop control operation parameters associated with the medication delivery and analyte sensor are performed to determine a predictive glucose response. Thereafter, the determined predictive glucose response is compared with the corresponding monitored analyte sensor signal and a sensor signal condition based on the comparison is determined. For example, based on the comparison, the sensor signal condition may indicate a signal attenuation condition of the glucose sensor.

SUMMARY

The invention provides a computer-implemented method as defined in claim 1 and an analyte monitoring system as defined in claim 7. Preferred embodiments of the invention are defined in the dependent claims.

The disclosure further includes a computer-implemented method for determining the existence of a potential event and causing a request for confirmation of the potential event to be displayed to the user; and treating first analyte data as more reliable than second analyte data if the potential event is confirmed by the user. In some aspects, the method further comprises determining a recommended insulin dose based upon analyte data received from a continuous analyte monitor. The analyte data is generated by an in vivo analyte sensor.

BRIEF DESCRIPTION OF THE DRAWINGS

FIG. 1 shows a data monitoring and management system such as, for example, an analyte (e.g., glucose) monitoring system in accordance with certain embodiments of the present disclosure;

FIG. 2 illustrates a data monitoring and management system for real time glucose measurement data acquisition and processing in one aspect of the present disclosure;

FIG. 3 is a block diagram of a receiver/monitor unit such as that shown in FIG. 1 in accordance with certain embodiments;

FIG. 4 is a flow chart illustrating a method for determining a recommended insulin dose based upon analyte data received from a continuous analyte monitor in accordance with certain embodiments of the present disclosure;

FIG. 5 is a graph depicting a percentage change to a recommended insulin dose based upon a glucose rate-of-change in accordance with certain embodiments of the present disclosure;

FIG. 6 is a flow chart illustrating a method for determining a recommended insulin dose based upon analyte data received from a continuous analyte monitor in accordance with certain embodiments of the present disclosure;

FIG. 7 is a flow chart illustrating a method for determining a recommended insulin dose based upon analyte data received from a continuous analyte monitor in accordance with certain embodiments of the present disclosure; and

FIG. 8 is a graph depicting trend-adjusted insulin correction based upon the glucose value or the glucose rate-of-change.

DETAILED DESCRIPTION

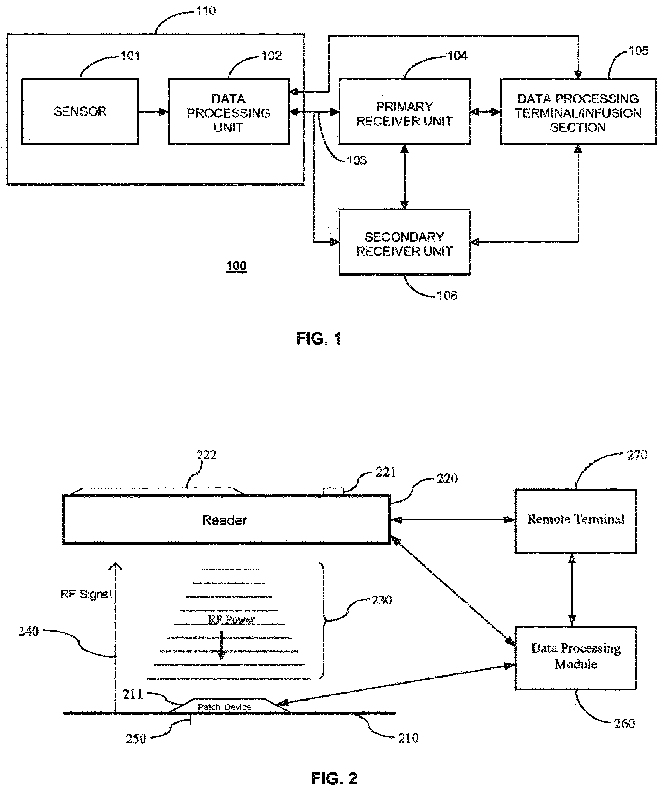

FIG. 1 shows a data monitoring and management system such as, for example, an analyte (e.g., glucose) monitoring system in accordance with certain embodiments of the present disclosure. Embodiments of the subject disclosure are described primarily with respect to glucose monitoring devices and systems, and methods of using two or more devices in a glucose monitoring system to reduce the likelihood of a failure of one or more of the devices in the glucose monitoring system going unnoticed by a user.

Analytes that may be monitored include, but are not limited to, acetyl choline, amylase, bilirubin, cholesterol, chorionic gonadotropin, creatine kinase (e.g., CK-MB), creatine, DNA, fructosamine, glucose, glutamine, growth hormones, hormones, ketones, lactate, peroxide, prostate-specific antigen, prothrombin, RNA, thyroid stimulating hormone, and troponin. The concentration of drugs, such as, for example, antibiotics (e.g., gentamicin, vancomycin, and the like), digitoxin, digoxin, drugs of abuse, theophylline, and warfarin, may also be monitored. In the embodiments that monitor more than one analyte, the analytes may be monitored at the same or different times.

Referring to FIG. 1, the analyte monitoring system 100 includes a sensor 101, a data processing unit (e.g., sensor electronics) 102 connectable to the sensor 101, and a primary receiver unit 104 which is configured to communicate with the data processing unit 102 via a communication link 103. In aspects of the present disclosure, the sensor 101 and the data processing unit (sensor electronics) 102 may be configured as a single integrated assembly 110. In certain embodiments, the integrated sensor and sensor electronics assembly 110 may be configured as an on-body patch device. In such embodiments, the on-body patch device may be configured for, for example, RFID or RF communication with a reader device/receiver unit, and/or an insulin pump.

In certain embodiments, the primary receiver unit 104 may be further configured to transmit data to a data processing terminal 105 to evaluate or otherwise process or format data received by the primary receiver unit 104. The data processing terminal 105 may be configured to receive data directly from the data processing unit 102 via a communication link which may optionally be configured for bi-directional communication. Further, the data processing unit 102 may include a transmitter or a transceiver to transmit and/or receive data to and/or from the primary receiver unit 104, the data processing terminal 105 or optionally the secondary receiver unit 106.

Also shown in FIG. 1 is an optional secondary receiver unit 106 which is operatively coupled to the communication link and configured to receive data transmitted from the data processing unit 102. The secondary receiver unit 106 may be configured to communicate with the primary receiver unit 104, as well as the data processing terminal 105. The secondary receiver unit 106 may be configured for bi-directional wireless communication with each of the primary receiver unit 104 and the data processing terminal 105. As discussed in further detail below, in certain embodiments the secondary receiver unit 106 may be a de-featured receiver as compared to the primary receiver unit 104, i.e., the secondary receiver unit 106 may include a limited or minimal number of functions and features as compared with the primary receiver unit 104. As such, the secondary receiver unit 106 may include a smaller (in one or more, including all, dimensions), compact housing or be embodied in a device such as a wrist watch, arm band, etc., for example. Alternatively, the secondary receiver unit 106 may be configured with the same or substantially similar functions and features as the primary receiver unit 104. The secondary receiver unit 106 may include a docking portion to be mated with a docking cradle unit for placement by, e.g., the bedside for night time monitoring, and/or bi-directional communication device.

Only one sensor 101, data processing unit 102 and data processing terminal 105 are shown in the embodiment of the analyte monitoring system 100 illustrated in FIG. 1. However, it will be appreciated by one of ordinary skill in the art that the analyte monitoring system 100 may include more than one sensor 101 and/or more than one data processing unit 102, and/or more than one data processing terminal 105.

The analyte monitoring system 100 may be a continuous monitoring system, or semi-continuous, or a discrete monitoring system. In a multi-component environment, each component may be configured to be uniquely identified by one or more of the other components in the system so that communication conflict may be readily resolved between the various components within the analyte monitoring system 100. For example, unique IDs, communication channels, and the like, may be used.

In certain embodiments, the sensor 101 is physically positioned in or on the body of a user whose analyte level is being monitored. The data processing unit 102 is coupleable to the sensor 101 so that both devices are positioned in or on the user's body, with at least a portion of the analyte sensor 101 positioned transcutaneously. The data processing unit 102 in certain embodiments may include a portion of the sensor 101 (proximal section of the sensor in electrical communication with the data processing unit 102) which is encapsulated within or on the printed circuit board of the data processing unit 102 with, for example, potting material or other protective material. The data processing unit 102 performs data processing functions, where such functions may include but are not limited to, filtering and encoding of data signals, each of which corresponds to a sampled analyte level of the user, for transmission to the primary receiver unit 104 via the communication link 103. In one embodiment, the sensor 101 or the data processing unit 102 or a combined sensor/data processing unit may be wholly implantable under the skin layer of the user.

In one aspect, the primary receiver unit 104 may include an analog interface section including an RF receiver and an antenna that is configured to communicate with the data processing unit 102 via the communication link 103, and a data processing section for processing the received data from the data processing unit 102 such as data decoding, error detection and correction, data clock generation, and/or data bit recovery.

In operation, the primary receiver unit 104 in certain embodiments is configured to synchronize with the data processing unit 102 to uniquely identify the data processing unit 102, based on, for example, an identification information of the data processing unit 102, and thereafter, to periodically receive signals transmitted from the data processing unit 102 associated with the monitored analyte levels detected by the sensor 101. That is, when operating in the CGM mode, the receiver unit 104 in certain embodiments is configured to automatically receive data related to the analyte level of a user from the analyte sensor/sensor electronics when the communication link (e.g., RF range) is maintained between these components.

Referring again to FIG. 1, the data processing terminal 105 may include a personal computer, portable data processing devices or computers such as a laptop computer or a handheld device (e.g., personal digital assistants (PDAs), communication devices such as a cellular phone (e.g., a multimedia and Internet-enabled mobile phone such as an iPhone, a Blackberry device, a Palm device such as Palm Pre, Treo, or similar phone), mp3 player, pager, and the like), drug delivery device, insulin pump, each of which may be configured for data communication with the receiver via a wired or a wireless connection. Additionally, the data processing terminal 105 may further be connected to a data network (not shown).

The data processing terminal 105 may include an infusion device such as an insulin infusion pump or the like, which may be configured to administer insulin to patients, and which may be configured to communicate with the primary receiver unit 104 for receiving, among others, the measured analyte level. Alternatively, the primary receiver unit 104 may be configured to integrate an infusion device therein so that the primary receiver unit 104 is configured to administer insulin (or other appropriate drug) therapy to patients, for example, for administering and modifying basal profiles, as well as for determining appropriate boluses for administration based on, among others, the detected analyte levels received from the data processing unit 102. An infusion device may be an external device or an internal device (wholly implantable in a user). An insulin bolus calculator may be operatively coupled to the primary receiver unit 104 to determine an insulin dose that is required based upon the analyte data received from the sensor device/electronics.

In particular embodiments, the data processing terminal 105, which may include an insulin pump, may be configured to receive the analyte signals from the data processing unit 102, and thus, incorporate the functions of the primary receiver unit 104 including data processing for managing the patient's insulin therapy and analyte monitoring. In certain embodiments, the communication link 103 as well as one or more of the other communication interfaces shown in FIG. 1 may use one or more of an RF communication protocol, an infrared communication protocol, a Bluetooth enabled communication protocol, an 802.11x wireless communication protocol, or an equivalent wireless communication protocol which would allow secure, wireless communication of several units (for example, per HIPAA requirements) while avoiding potential data collision and interference.

As described in aspects of the present disclosure, the analyte monitoring system may include an on-body patch device with a thin profile that can be worn on the arm or other locations on the body (and under clothing worn by the user or the patient), the on-body patch device including an analyte sensor and circuitry and components for operating the sensor and processing and storing signals, received from the sensor as well as for communication with the reader device. For example, one aspect of the on-body patch device may include electronics to sample the voltage signal received from the analyte sensor in fluid contact with the body fluid, and to process the sampled voltage signals into the corresponding glucose values and/or store the sampled voltage signal as raw data, and to process the raw data.

In certain embodiments, the on-body patch device includes an antenna such as a loop antenna to receive RF power from an external device such as the reader device/receiver unit described above, electronics to convert the RF power received via the antenna into DC (direct current) power for the on-body patch device circuitry, communication module or electronics to detect commands received from the reader device, and communication component to transmit data to the reader device, a low capacity battery for providing power to sensor sampling circuitry (for example, the analog front end circuitry of the on-body patch device in signal communication with the analyte sensor), one or more non-volatile memory or storage device to store data including raw signals from the sensor or processed data based on the raw sensor signals. More specifically, in the on operation demand mode, the on body patch device in certain embodiments is configured to transmit real time analyte related data and/or stored historical analyte related data, and/or functionality data when within the RF power range of the reader device. As such, when the reader device is removed or positioned out of range relative to the on body patch device, the on body patch device may no longer transmit the analyte related data.

In certain embodiments, a data processing module/terminal may be provided in the analyte monitoring system that is configured to operate as a data logger, interacting or communicating with the on-body patch device by, for example, transmitting requests for information to the on-body patch device, and storing the responsive information received from the on-body patch device in one or more memory components of the data processing module (e.g., repeater unit). Further, data processing module may be configured as a compact on-body relay device to relay or retransmit the received analyte level information from the on-body patch device to the reader device/receiver unit or the remote terminal or both. The data processing module in one aspect may be physically coupled to the on-body patch device, for example, on a single adhesive patch on the skin surface of the patient. Alternatively, the data processing module may be positioned close to but not in contact with the on-body patch device. For example, when the on-body patch device is positioned on the abdomen of the patient, the data processing module may be worn on a belt of the patient or the user, such that the desired close proximity or predetermined distance of approximately 2,54 - 12.7 cm (1-5 inches), or about 2.54 - 25.4 cm (1-10 inches), for example, or more, between the on-body patch device and the data processing module may be maintained.

The various processes described above including the processes operating in the software application execution environment in the analyte monitoring system including the on-body patch device, the reader device, data processing module and/or the remote terminal performing one or more routines described above may be embodied as computer programs developed using an object oriented language that allows the modeling of complex systems with modular objects to create abstractions that are representative of real world, physical objects and their interrelationships. The software required to carry out the inventive process, which may be stored in a memory or storage device of the storage unit of the various components of the analyte monitoring system described above in conjunction to the Figures including the on-body patch device, the reader device, the data processing module, various described communication devices, or the remote terminal may be developed by a person of ordinary skill in the art and may include one or more computer program products.

In one embodiment, an apparatus for bi-directional communication with an analyte monitoring system may comprise a storage device having stored therein one or more routines, a processing unit operatively coupled to the storage device and configured to retrieve the stored one or more routines for execution, a data transmission component operatively coupled to the processing unit and configured to transmit data based at least in part on the one or more routines executed by the processing unit, and a data reception component operatively coupled to the processing unit and configured to receive analyte related data from a remote location and to store the received analyte related data in the storage device for retransmission, wherein the data transmission component is programmed to transmit a query to a remote location, and further wherein the data reception component receives the analyte related data from the remote location in response to the transmitted query when one or more electronics in the remote location transitions from an inactive state to an active state upon detection of the query from the data transmission component.

FIG. 2 illustrates a data monitoring and management system for analyte related data acquisition and processing in one aspect of the present disclosure. More specifically, as shown in FIG. 2, the on-body patch device 211 including sensor electronics coupled to an analyte sensor 250 is positioned on a skin surface 210 of a patient or a user.

Referring back to FIG. 2, as shown, when the reader device/receiver unit 220 is positioned or placed in close proximity and within a predetermined range of the on-body patch device 211, the RF power supply in the reader device/receiver unit 220 may be configured to provide the necessary power to operate the electronics in the on-body patch device 211, and the on-body patch device 211 may be configured to, upon detection of the RF power from the reader device/receiver unit 220, perform preprogrammed routines including, for example, transmitting one or more signals 240 to the reader device/receiver unit 220 indicative of the analyte level of the user.

In certain embodiments, the reader device/receiver unit 220 may include an RF power switch that is user activatable or activated upon positioning within a predetermined distance from the on body patch device 211 to turn on the analyte sensor in the on body patch device 211. That is, using the RF signal, the analyte sensor coupled to the sensor electronics in the on-body patch device 211 may be initialized or activated. In another embodiment, a passive RFID function may be provided or programmed such that upon receiving a "turn on" signal which, when authenticated, will turn on the electronic power switch that activates the on-body patch device 211. That is, the passive RFID configuration may include drawing energy from the RF field radiated from the reader device/receiver unit 220 so as to prompt for and/or detect the "turn on" signal which, upon authentication, activates the on body patch device 211.

As further shown in FIG. 2, the display 222 of the reader device/receiver unit 220 may be configured to provide the functionalities of a user interface to present information such as alarm or alert notification to the user. In one aspect, the reader device/receiver unit 220 may include other output components such as a speaker, vibratory output component and the like to provide audible and/or vibratory output indication to the user in addition to the visual output indication provided on the display 222.

As discussed, some or all of the electronics in the on-body patch device 211 in one embodiment may be configured to rely on the RF power received from the reader device/receiver unit 220 to perform analyte data processing and/or transmission of the processed analyte information to the reader device/receiver unit 220. That is, the on-body patch device 211 may be discreetly worn on the body of the user or the patient, and under clothing, for example, and when desired, by positioning the reader device/receiver unit 220 within a predetermined distance from the on-body patch device 211, analyte information may be received by the reader device/receiver unit 220.

Referring still to FIG. 2, also shown are a data processing module/terminal 260 and a remote terminal 270. In one aspect, data processing module 260 may include a stand alone device configured for bi-directional communication to communicate with the on-body patch device 211, the reader device/receiver unit 220 and/or the remote terminal 270. More specifically, data processing module 260 may include one or more microprocessors or similar data processing components configured to execute one or more software routines for communication, as well as data storage and retrieval to and from one or more memory components provided in the housing of the data processing module 260.

The data processing module 260 in one embodiment may be configured to communicate with the on-body patch device 211 in a similar manner as the reader device/receiver unit 220 and may include communication components such as antenna, power supply and memory, among others, for example, to allow provision of RF power to the on-body patch device 211 or to request or prompt the on-body patch device 211 to send the analyte related data and optionally stored analyte related data. The data processing module 260 may be configured to interact with the on-body patch device 211 in a similar manner as the reader device/receiver unit 220 such that the data processing module 260 may be positioned within a predetermined distance from the on-body patch device 211 for communication with the on-body patch device 211.

In one aspect, the on-body patch device 211 and the data processing module 260 may be positioned on the skin surface of the user or the patient within the predetermined distance of each other (for example, within approximately 12.7 cm (5 inches) or less) such that the communication between the on-body patch device 211 and the data processing module 260 is maintained. In a further aspect, the housing of the data processing module 260 may be configured to couple to or cooperate with the housing of the on-body patch device 211 such that the two devices are combined or integrated as a single assembly and positioned on the skin surface.

Referring again to FIG. 2, the data processing module 260 may be configured or programmed to prompt or ping the on-body patch device 211 at a predetermined time interval such as once every minute, or once every five minutes or once every 30 minutes or any other suitable or desired programmable time interval to request analyte related data from the on-body patch device 211 which is received and is stored in one or more memory devices or components of the data processing module 260. In another embodiment, the data processing module 260 is configured to prompt or ping the on-body patch device 211 when desired by the patient or the user on-demand, and not based on a predetermined time interval. In yet another embodiment, the data processing module 260 is configured to prompt or ping the on-body patch device 211 when desired by the patient or the user upon request only after a programmable time interval has elapsed. For example, in certain embodiments, if the user does not initiate communication within a programmed time period, such as, for example 5 hours from last communication (or 10 hours from the last communication), the data processing module 260 may be programmed to automatically ping or prompt the on-body patch device 211 or alternatively, initiate an alarm function to notify the user that an extended period of time has elapsed since the last communication between the data processing module 260 and the on-body patch device 211. In this manner, users, healthcare providers, or the patient may program or configure the data processing module 260 to provide certain compliance with analyte monitoring regimen, to avoid a failure of the analyte sensor device from going unnoticed. Similar functionalities may be provided or programmed in the receiver unit or the reader device in certain embodiments.

As further shown in FIG. 2, the data processing module 260 in one aspect may be configured to transmit the stored data received from the on-body patch device 211 to the reader device/receiver unit 220 when communication between the data processing module 260 and the reader device/receiver unit 220 is established. More specifically, in addition to RF antenna and RF communication components described above, data processing module 260 may include components to communicate using one or more wireless communication protocols such as, for example, but not limited to, infrared (IR) protocol, Bluetooth protocol, Zigbee protocol, and 802.11 wireless LAN protocol. Additional description of communication protocols including those based on Bluetooth protocol and/or Zigbee protocol can be found in U.S. Patent Publication No. 2006/0193375. The data processing module 260 may further include communication ports, drivers or connectors to establish wired communication with one or more of the reader device/receiver unit 220, on-body patch device 211, or the remote terminal 270 including, for example, but not limited to USB connector and/or USB port, Ethernet connector and/or port, Fire Wire connector and/or port, or RS-232 port and/or connector.

In one aspect, the data processing module 260 may be configured to operate as a data logger configured or programmed to periodically request or prompt the on-body patch device 211 to transmit the analyte related information, and to store the received information for later retrieval or subsequent transmission to the reader device/receiver unit 220 or to the remote terminal 270 or both, for further processing and analysis.

In a further aspect, the functionalities of the data processing module 260 may be configured or incorporated into a memory device such as an SD card, microSD card, compact flash card, XD card, Memory Stick card, Memory Stick Duo card, or USB memory stick/device including software programming resident in such devices to execute upon connection to the respective one or more of the on-body patch device 211, the remote terminal 270 or the reader device/receiver unit 220. In a further aspect, the functionalities of the data processing module 260, including executable software and programming, may be provided to a communication device such as a mobile telephone including, for example, iPhone, iTouch, Blackberry device, Palm based device (such as Palm Pre, Treo, Treo Pro, Centra), personal digital assistants (PDAs) or any other communication enabled operating system (such as Windows or Android operating systems) based mobile telephones as a downloadable application for execution by the downloading communication device. To this end, the remote terminal 270 as shown in FIG. 2 may include a personal computer, or a server terminal that is configured to provide the executable application software to the one or more of the communication devices described above when communication between the remote terminal 270 and the devices are established.

Depending upon the user setting or configuration on the communication device, the downloaded application may be programmed or customized using the user interface of the respective communication device (screen, keypad, and the like) to establish or program the desired settings such as a receiver alarm, an insulin pump alarm, sensor replacement alarm, or any other alarm or alert conditions as may be desired by the user. Moreover, the programmed notification settings on the communication device may be output using the output components of the respective communication devices, such as speaker, vibratory output component, or visual output/display. As a further example, the communication device may be provided with programming and application software to communicate with the on-body patch device 211 such that a frequency or periodicity of data acquisition is established. In this manner, the communication device may be configured to conveniently receive analyte information from the on-body patch device 211 at predetermined time periods such as, for example, but not limited to once every minute, once every five minutes, or once every 10 or 15 minutes, and store the received information, as well as to provide a desired or appropriate warning indication or notification to the user or the patient.

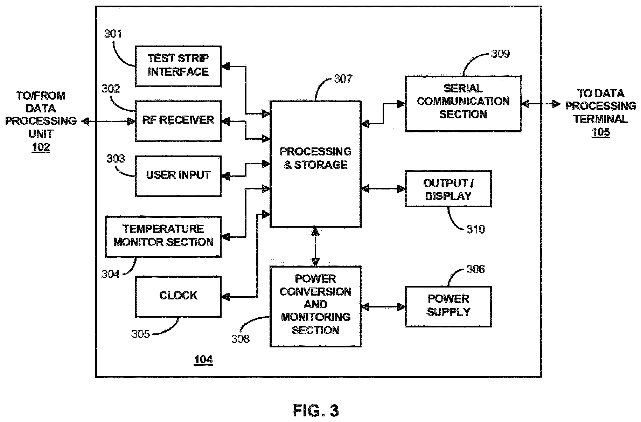

FIG. 3 is a block diagram of a receiver/monitor unit or insulin pump such as that shown in FIG. 1 in accordance with certain embodiments. The primary receiver unit 104 (FIG. 1) includes one or more of: a blood glucose test strip interface 301, an RF receiver 302, an input 303, a temperature detection section 304, and a clock 305, each of which is operatively coupled to a processing and storage section 307. The primary receiver unit 104 also includes a power supply 306 operatively coupled to a power conversion and monitoring section 308. Further, the power conversion and monitoring section 308 is also coupled to the receiver processor 307. Moreover, also shown are a receiver serial communication section 309, and an output 310, each operatively coupled to the processing and storage unit 307. The receiver may include user input and/or interface components or may be free of user input and/or interface components.

In one aspect, the RF receiver 302 is configured to communicate, via the communication link 103 (FIG. 1) with the data processing unit (sensor electronics) 102, to receive encoded data from the data processing unit 102 for, among others, signal mixing, demodulation, and other data processing. The input 303 of the primary receiver unit 104 is configured to allow the user to enter information into the primary receiver unit 104 as needed. In one aspect, the input 303 may include keys of a keypad, a touch-sensitive screen, and/or a voice-activated input command unit, and the like. The temperature monitor section 304 may be configured to provide temperature information of the primary receiver unit 104 to the processing and storage section 307, while the clock 305 provides, among others, real time or clock information to the processing and storage section 307.

Each of the various components of the primary receiver unit 104 shown in FIG. 3 is powered by the power supply 306 (or other power supply) which, in certain embodiments, includes a battery. Furthermore, the power conversion and monitoring section 308 is configured to monitor the power usage by the various components in the primary receiver unit 104 for effective power management and may alert the user, for example, in the event of power usage which renders the primary receiver unit 104 in sub-optimal operating conditions. The serial communication section 309 in the primary receiver unit 104 is configured to provide a bi-directional communication path from the testing and/or manufacturing equipment for, among others, initialization, testing, and configuration of the primary receiver unit 104.

Serial communication section 309 can also be used to upload data to a computer, such as functionality related data. The communication link with an external device (not shown) can be made, for example, by cable (such as USB or serial cable), infrared (IR) or RF link. The output/display 310 of the primary receiver unit 104 is configured to provide, among others, a graphical user interface (GUI), and may include a liquid crystal display (LCD) for displaying information. Additionally, the output/display 310 may also include an integrated speaker for outputting audible signals as well as to provide vibration output as commonly found in handheld electronic devices, such as mobile telephones, pagers, etc. In certain embodiments, the primary receiver unit 104 also includes an electro-luminescent lamp configured to provide backlighting to the output 310 for output visual display in dark ambient surroundings.

Referring back to FIG. 3, the primary receiver unit 104 may also include a storage section such as a programmable, non-volatile memory device as part of the processor 307, or provided separately in the primary receiver unit 104, operatively coupled to the processor 307. The processor 307 may be configured to perform Manchester decoding (or other protocol(s)) as well as error detection and correction upon the encoded data received from the data processing unit 102 via the communication link 103.

In further embodiments, the data processing unit 102 and/or the primary receiver unit 104 and/or the secondary receiver unit 106, and/or the data processing terminal/infusion section 105 may be configured to receive the blood glucose value wirelessly over a communication link from, for example, a blood glucose meter. In further embodiments, a user manipulating or using the analyte monitoring system 100 (FIG. 1) may manually input the blood glucose value using, for example, a user interface (for example a keyboard, keypad, voice commands, and the like) incorporated in the one or more of the data processing unit 102, the primary receiver unit 104, secondary receiver unit 106, or the data processing terminal/infusion section 105.

Additional detailed descriptions are provided in U.S. Patent Nos. 5,262,035; 5,264,104; 5,262,305; 5,320,715; 5,593,852; 6,175,752; 6,650,471; 6,746, 582, 6,284,478, 7,299,082, and in application No. 10/745,878 filed December 26, 2003 titled "Continuous Glucose Monitoring System and Methods of Use", in application No. 11/060,365 filed February 16, 2005 titled "Method and System for Providing Data Communication in Continuous Glucose Monitoring And Management System", and in application No. 12/698,124 filed February 1, 2010 titled "Compact On-Body Physiological Monitoring Devices and Methods thereof,".

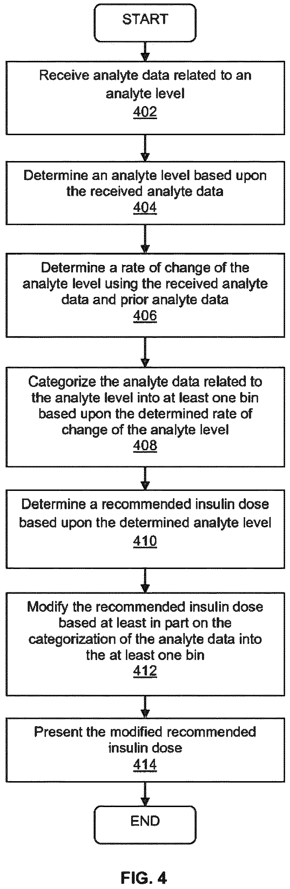

FIG. 4 is a flow diagram illustrating steps in an embodiment for determining an insulin dosage recommendation based at least upon analyte related data received from an analyte sensor device in an analyte monitoring system. The embodiment can provide a means to receive an insulin dosage recommendation (e.g., at a receiver device or an insulin pump) based upon data that is related to a user's current analyte level, target glucose level, insulin sensitivity, insulin-to-carbohydrate ratio, insulin-on-board, and meal size. Analyte data related to an analyte level of a user can be transmitted from an analyte monitoring device to a receiver device in the analyte monitoring system (402). The request can be sent, for example, wirelessly from the transmitter of the analyte monitoring device to the transceiver of the receiver device. The receiver device can determine an analyte level of the user based upon the received analyte data (404). A rate of change of the analyte level of the user can be determined by the receiver device, based upon one or more of the received analyte data and prior analyte data (406). The prior analyte data can, for example, be stored in one or more processors in the receiver device, or at an external location that can be in communication with the receiver device.

Still referring to FIG. 4, the receiver device can categorize the analyte data into at least one bin that corresponds to the determined rate of change of the analyte level (408). A recommended insulin dose can be determined by the receiver device based upon the determined analyte level of the user (410). The receiver device can modify the recommended insulin dose based at least in part on the categorization of the analyte data into the at least one bin (412). The receiver device can also present the modified recommended insulin dose to the user (414). The modified recommended insulin dose can be presented to the user, for example, in the form of an alpha-numeric display, a graphical display, and an audible display. Moreover, the modified recommended insulin dose can be transmitted to an insulin administration device (e.g., an insulin pump, an insulin pen, and an insulin patch), for automatic administration of insulin to the user.

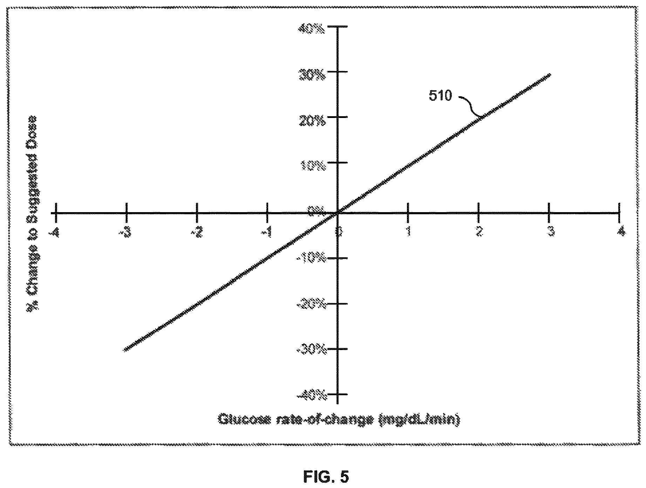

As illustrated in FIG. 5, insulin recommendations based upon the categorization of analyte sensor data into a corresponding bin based upon the rate-of-change bin (e.g., trend arrow), and the following correction could be applied:

- analyte rates of change ≥ 2 mg/dL/min, increase recommended dose by 20%;
- analyte rates of change ≥ 1 mg/dL/min and < 2 mg/dL/min, increase recommended dose by 10%;
- analyte rates of change ≥ -1 mg/dL/min and < 1 mg/dL/min (or no trend information available), do not modify recommended dose;
- analyte rates of change ≥ -2 mg/dL/min and < -1 mg/dL/min, decrease recommended dose by 10%; and
- analyte rates of change ≤ -2 mg/dL/min, decrease recommended dose by 20%.

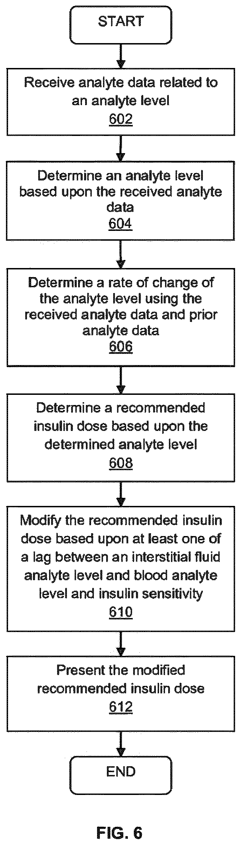

FIG. 6 is a flow diagram illustrating certain embodiments for determining an insulin dosage recommendation based at least upon analyte related data received from an analyte sensor device in an analyte monitoring system. The embodiment can provide a means to receive an insulin dosage recommendation (e.g., at a receiver device or an insulin pump) based upon data that is related to a user's current analyte level, target glucose level, insulin sensitivity, insulin-to-carbohydrate ratio, insulin-on-board, and meal size. Analyte data related to an analyte level of a user can be transmitted from an analyte monitoring device to a receiver device in the analyte monitoring system (602). The request can be sent, for example, wirelessly from the transmitter of the analyte monitoring device to the transceiver of the receiver device. The receiver device can determine an analyte level of the user based upon the received analyte data (604). A rate of change of the analyte level of the user can be determined, by the device, based upon one or more of the received analyte data and prior analyte data (606). The prior analyte data can, for example, be stored in one or more processors in the receiver device, or at an external location.

Still referring to FIG. 6, a recommended insulin dose can be determined by the receiver device based upon the determined analyte level of the user (608). The recommended insulin dose can be modified based upon at least one of a lag between an interstitial analyte level determined by the sensor and the actual blood analyte level, and the user's insulin sensitivity (610). The lag between the interstitial analyte level and the blood analyte level can be determined based upon a calibration factor that is associated with the analyte sensor and is determined by the sensor manufacturer. Alternately, the lag calibration factor can be determined by taking an analyte reading of the interstitial fluid using the sensor, and then prompting the user for a blood analyte reading, for example using a finger-stick method. The user's insulin sensitivity can be determined by a healthcare provider, or alternately, by the analyte monitoring system. The receiver device can also present the modified recommended insulin dose to the user (612). The modified recommended insulin dose can be presented to the user, for example, in the form of an alpha-numeric display, a graphical display, and an audible display. Moreover, the modified recommended insulin dose can be transmitter to an insulin administration device (e.g., an insulin pump, an insulin pen, and an insulin patch), for automatic administration of insulin to the user.

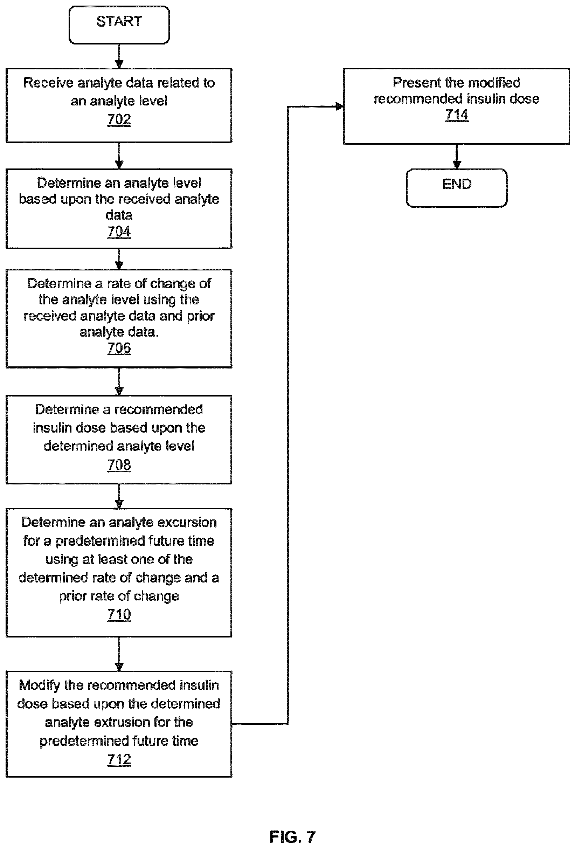

FIG. 7 is a flow diagram illustrating steps in an embodiment for determining an insulin dosage recommendation based at least upon analyte related data received from an analyte sensor device in an analyte monitoring system. The embodiment can provide a means to receive an insulin dosage recommendation (e.g., at a receiver device or an insulin pump) based upon data that is related to a user's current analyte level, target glucose level, insulin sensitivity, insulin-to-carbohydrate ratio, insulin-on-board, and meal size. Analyte data related to an analyte level of a user can be transmitted from an analyte monitoring device to a receiver device in the analyte monitoring system (702). The request can be sent, for example, wirelessly from the transmitter of the analyte monitoring device to the transceiver of the receiver device. The receiver device can determine an analyte level of the user based upon the received analyte data (704). A rate of change of the analyte level of the user can be determined, by the device, based upon one or more of the received analyte data and prior analyte data (706). The prior analyte data can, for example, be stored in one or more processors in the receiver device, or at an external location.

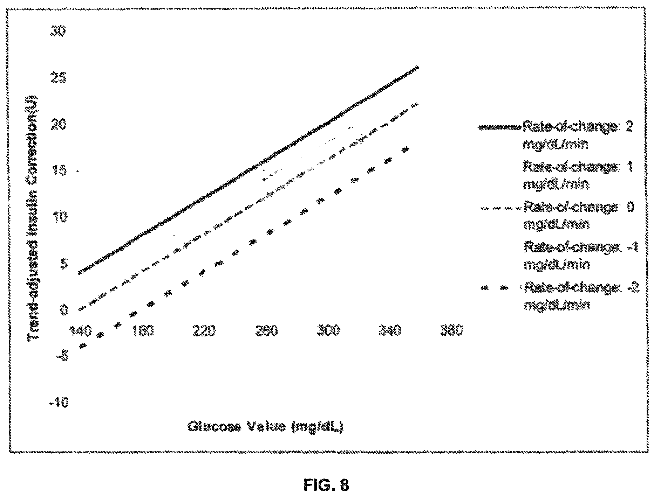

Still referring to FIG. 7, a recommended insulin dose can be determined by the receiver device based upon the determined analyte level of the user (708). The receiver device can determine an analyte excursion for a predetermined future time using at least one of the determined rate of change and a prior rate of change (710). For example, the analyte excursion can be determined using the received analyte data, the determined rate-of-change of the analyte level, the user's insulin sensitivity and the user's target analyte level. By further way of example, a user with an insulin sensitivity factor (ISF) of 10 (1 unit of insulin drops the user's blood glucose level by 10 mg/dL) and a target value of 140 mg/dL, and the user is attempting to correct for the expected change in glucose over the next 20 minute could have a correction dose as: ((current glucose + trend*20 minutes) - target)/(interstitial glucose level), which is illustrated in FIG. 8.

Referring to FIG. 7, the receiver device can also present the modified recommended insulin dose to the user (712), for example, in the form of an alpha-numeric display, a graphical display, and an audible display. Moreover, the modified recommended insulin dose can be transmitter to an insulin administration device (e.g., an insulin pump, an insulin pen, an insulin patch, and an aerosol insulin device), for automatic administration of insulin to the user.

Moreover, the implementation of the bolus calculator as described above can be impacted by the possible inclusion or exclusion of an analyte strip port and by the expected conditional replacement of sensor-based analyte measurements. The bolus calculator can allow the user to enter analyte measurements that are acquired by an analyte test strip, if no strip port is present in the receiver unit.

The bolus calculator may be pre-populated by sensor analyte readings when the condition for the replacement of the sensor is required. In this instance, the user may be allowed to manually override the pre-populated sensor analyte value or a strip analyte value within a predetermined amount of time (e.g., 5 minutes) may automatically be used to override the sensor analyte value. If manually overridden, the system should interpret this value as a strip analyte measurement, and an indication to the user (e.g., blood drop icon) on the display of the receiver device. The system may or may not still incorporate a rate-of-change correction based upon the sensor-derived rate-of-change.

If the user is experiencing conditions of a low blood analyte level (e.g., if this is one of the conditions where the replacement condition is not met) the analyte monitoring system may not allow a correction dose of insulin, but may still allow a meal dose of insulin based upon the user entered meal size. Alternately, the system may allow reverse correction of a low analyte level (e.g., subtract low analyte correction from the meal insulin).

By way of another example, when the user is experiencing high rates of change in the blood analyte level (e.g., if this is one of the conditions where the replacement condition is not met) the analyte monitoring system may require a strip glucose value that can be entered by the user or determined directly from a test strip if a strip port is integrated in the receiver device.

User configurable settings can include "trend sensitivity" (e.g., the percentage correction based upon the analyte rate of change bins or alternatively, the correction equation of the estimate of lag time between interstitial analyte level and the blood analyte level). The trend sensitivity may be configured by a healthcare professional, similar to an insulin sensitivity or insulin-to-carbohydrate ratio.

Additionally, factory configurable settings include limits of conditional replacement of the analyte readings, whether the bolus calculator is enabled or disabled, and whether the bolus calculator may only be enabled by a healthcare entered passcode or other such approach to limit distribution.

According to an embodiment of the claimed invention, when a segment of analyte data is available (e.g. an 8 hour span of glucose data recorded at 15 minute intervals made available when a user queries the on-demand system), a mathematical model of the user's analyte level can be reconciled against this data. For example, an autoregressive (AR) model of the form: $g(k) = (a1\ z(k-1)) + (a2\ z(k-2)) + ... + (aN\ z(k-N))$ can be adopted. Where $g(k)$ is the estimated analyte value at sample time k. Such an AR model can assume that at any instance, a signal estimate can be obtained strictly from a weighted sum of a measurement source z at different sample times up to the present sample time *k*. The constants *a1, a2, ... , aN* may either be determined *a priori* (e.g., from population data) or allowed to vary over time based on an adaptive rule. The size of the model can determine the value of *N*.

The estimate $g(k)$ is compared to the measurement at the same instance, $z(k)$. Different metrics can be derived to reflect this comparison. For example, the simplest metric is the absolute difference: $I(k)=g(k)-z(k)$. Other metrics include a moving average version of the absolute difference, or combinations with time derivatives of the difference. Whenever the metric exceeds a certain threshold, at least one of the 2 things may have occurred. The first is that there is an external influence that dramatically changes the course of a user's analyte level, such as meals, insulin, and exercise. The second is that sensor artifacts contaminate one or more measurements z within the *N* sample time window.

The former can be an opportunity to enrich the analyte data with event tagging around sample instances where the chosen metric exceeds a given threshold. The latter is an opportunity to improve analyte data integrity by either discounting certain glucose sections or attempting to repair that segment. Dropout detection and compensation methods such as described in U.S. Patent No. 7,630,748 can be applied to that segment.

A more elaborate model that includes insulin and meal related states can also be used. One example is the extension of the Bergman minimal model, which is described in "Physiological Evaluation of Factors Controlling Glucose Tolerance in Man, Measurement of Insulin Sensitivity and Beta-cell Glucose Sensitivity From the Response to Intravenous Glucose", J. Clin. Invest., The American Society for Clinical Investigation, Inc., vol. 68, pp. 1456-1467, December 1981, more commonly written in the continuous time domain than in the above AR structure's discrete time domain:

- $$d/dt\ g(t) = ((p1 + SI\ X(t))\ g(t)) + (p1\ Gb) + (p3\ um(t))$$

- $$d/dt\ X(t) = p2\ (I(1) - Ib - X(t))$$

- $$d/dt\ I(l) = -(p4\ I) + ui(t)$$

Where the glucose, effective insulin, and plasma insulin states g, X, and *I* are estimated and tracked over time *I*. The presence of meals and insulin, *um* and *ui,* over time are considered as unknown disturbances to the system. The physiological parameters *pl, pl, pJ, p4, SI,* as well as the steady state glucose and insulin values *Gb* and *Ib,* may be set using prior population data, or set and then allowed to adapt over time using an appropriate adaptation algorithm.

A state observer such as a Kalman filter could then be employed to estimate the states. Then, using a similar comparative metric such as described for the AR case, any time a metric exceeds its threshold implies an external influence and/or a sensor artifact. In the state observer case, an estimate of the unknown disturbances can also be computed, allowing for a more reliable determination of whether a meal and/or insulin input has taken place. This is conceptually similar to the Disturbance Observer structure described by Umeno et al, "Robust Speed Control of DC Servomotors using Modern Two-Degrees- of-Freedom Controller Design", IEEE Trans, on Industrial Electronics, Vol. 38, No. 5, pp. 363-368, 1991.

According to the claimed invention, the estimation of an external event (e.g., via the AR, state observer, or other methods) is further enhanced by considering prior information of the user. For example, past meal, insulin, and exercise tags by the patient can be used to fit a statistical model that can track past events to determine most likely values of present events. For example, the model described by Winters in "Forecasting Sales by Exponentially Weighted Moving Averages," MANAGEMENT SCIENCE, vol. 6, pp. 324-342, April 1, 1960, can be applied to estimate the most likely timing and amount of the user's dinner meal based on the user's past logged dinner events. Combined, the two methods can increase the confidence that a certain event may be taking place at any instance glucose data is available. The next module, the non- intrusive tagging system, uses this to encourage confirmation from the patient. Analyte data obtained around confirmed events will be treated with a higher credibility by any treatment assessment algorithm, and will not go through any artifact detection/rejection mechanism.

Whenever historical glucose data is available to the system, and when the event estimator previously described estimates the presence of an event, a status icon (e.g. a question mark icon, or icons that represent the most likely event as estimated by the estimator) may appear in the main menu in the handheld display. Alternatively, an LED or the strip port could light up in a special color to attract the user's attention or the device could present an auditory and/or vibratory alert to the user that a potential event has been detected. The event estimator could be designed to present estimates to the user for review when requested by the user (e.g., when the feature is selected by the patient from the device UI) or the event estimator could be part of, and run from, a separate PC-based data management system. Should the user choose to respond by tapping on the on-screen icon or an alternate softkey, then a brief question could be provided, in which the user can confirm by choosing yes or no. For example, a query "Tap if you had breakfast around 8: 15am" accompanied by a historical glucose graph, could appear on screen. Tapping the screen confirms the event; sliding the screen allows for adjustment; ignoring the menu item after a pre-determined time removes the query.

In certain embodiments, the analyte monitoring system can include one or more projected analyte alarms based upon a linear extrapolation of the recent glucose history. This projection may be of a fixed time period (e.g., 10 minutes or 30 minutes) or may be configurable to the user (e.g., 10 minutes, 20 minutes, and/or 30 minutes). The threshold analyte level which, when projected to be crossed within the fixed time period triggers the alarm to annunciate, which can either be configured by the user or set as a default. Thresholds can exist for both adversely low and adversely high analyte levels.

The analyte monitoring system can request (e.g., send a reminder) that the user place the receiver device within a predetermined range of the analyte sensor device so that analyte sensor data can be transmitted to the receiver device, wherein the analyte sensor data is processed to determine if the predetermined threshold has been crossed. The reminder may be sent to the user at various times:

- a fixed time after the projected alarm (e.g., 30 minutes);
- a user configured time after the projected alarm (e.g., 45 minutes);
- at a time based on the receiver device's projection that the analyte value would have crossed the threshold analyte level (e.g., 23 minutes); and/or
- at a time equal to the predetermined time period of the configurable projected alarm (e.g., 10 minutes, 20 minutes, and/or 30 minutes).

Upon presenting the projected alarm, the user may or may not be asked by the user-interface of the receiver device if the user would like to receive a subsequent reminder to place the receiver device within a predetermined range of the analyte sensor device. This feature may optionally be configured as a general receiver setting.

In the manner provided, in certain embodiments, there is provided a computer-implemented method comprising receiving, at one or more processors, analyte data related to an analyte level of a user from a continuous analyte monitor, determining, using the one or more processors, an analyte level of the user based upon the received analyte data, determining a rate of change of the analyte level of the user using the received analyte data and prior analyte data, determining, using the one or more processors, a recommended insulin dose based upon the determined analyte level of the user, and modifying, using the one or more processors, the recommended insulin dose based upon at least one of a lag between an interstitial fluid analyte level and blood analyte level and the user's insulin sensitivity.

In certain embodiments, the computer-implemented method includes presenting, using the one or more processors, one or both of the recommended insulin dose and the modified recommended insulin dose to the user.

In certain embodiments, the computer-implemented method further comprises categorizing, using the one or more processors, the analyte data related to the analyte level of the user into at least one bin that corresponds to the determined rate of change of the analyte level.

In certain embodiments, the computer-implemented method includes modifying, using the one or more processors, the recommended insulin dose based at least in part on the categorization of the analyte data into the at least one bin.

In certain embodiments, the modified recommended insulin dose includes increasing the recommended insulin dose by at least 20% if the rate of change of the analyte level is ≥ 2 mg/dL/min.

In certain embodiments, the modified recommended insulin dose includes increasing the recommended insulin dose by at least 10% if the rate of change of the analyte level is determined to be ≥ 1 mg/dL/min and < 2 mg/dL/min.

In certain embodiments, the modified recommended insulin dose includes not modifying the recommended insulin dose if the analyte rate of change is determined to be ≥ -1 mg/dL/min and < 1 mg/dL/min or if a rate of change of the analyte level cannot be determined.

In certain embodiments, the computer-implemented method the modified recommended insulin dose includes decreasing the recommended insulin dose by at least 10% if the rate of change of the analyte level is determined to be ≥ -2 md/dL/min and < -1 mg/dL/min.

In certain embodiments, the modified recommended insulin dose includes decreasing the recommended insulin dose by at least 20% if the determined rate of change of the analyte level is ≥ -2mg/dL/min.

In certain embodiments, the computer-implemented method includes determining, using the one or more processors, an analyte excursion for a predetermined future time using at least one of the determined rate of change and a prior rate of change, modifying, using the one or more processors, the recommended insulin dose based upon the determined analyte excursion for the predetermined future time, presenting, using the one or more processors, the modified recommended insulin dose to the user.

In certain embodiments, the computer-implemented method includes determining, using the one or more processors, an analyte excursion for a predetermined future time using at least one of the received analyte data, the determined rate of change, an insulin sensitivity of the user, and an analyte level target value, modifying, using the one or more processors, the recommended insulin dose based upon the determined analyte extrusion for the predetermined future time, and presenting, using the one or more processors, the modified recommended insulin dose to the user.

In certain embodiments, the computer-implemented method includes transmitting, from the one or more processors, the modified recommended insulin dose to a drug administering device.

In certain embodiments, the lag is determined based upon a calibration factor associated with the analyte sensor.

In certain embodiments, the computer-implemented method includes modifying the recommended insulin dose based on trend sensitivity.

An apparatus in accordance with a further embodiment includes a user interface, one or more processors, and a memory storing instructions which, when executed by the one or more processors, causes the one or more processors to receive analyte data related to an analyte level of a user from a continuous analyte monitor, to determine an analyte level of the user based upon the received analyte data to determine a rate of change of the analyte level of the user using the received analyte data and prior analyte data, to determine a recommended insulin dose based upon the determined analyte level of the user, and to modify the recommended insulin dose based upon at least one of a lag between an interstitial fluid analyte level and blood analyte level and the user's insulin sensitivity.

In certain embodiments, the memory storing instructions which, when executed by the one or more processors, causes the one or more processors to present one or both of the recommended insulin dose and the modified recommended insulin dose on the user interface.

In certain embodiments, the memory storing instructions which, when executed by the one or more processors, causes the one or more processors to categorize the analyte data related to the analyte level of the user into at least one bin that corresponds to the determined rate of change of the analyte level.

In certain embodiments, the memory storing instructions which, when executed by the one or more processors, causes the one or more processors to modify the recommended insulin dose based at least in part on the categorization of the analyte data into the at least one bin.

In certain embodiments, the memory storing instructions which, when executed by the one or more processors, causes the one or more processors to determine an analyte excursion for a predetermined future time using at least one of the determined rate of change and a prior rate of change, to modify the recommended insulin dose based upon the determined analyte excursion for the predetermined future time, and to present the modified recommended insulin dose on the user interface.

**Claims**

1. A computer-implemented method comprising:

   receiving, by a processor (307) of an analyte monitoring system (104, 220), first analyte data generated by an in vivo analyte sensor (110, 211) at a sample time, wherein the in vivo analyte sensor is in contact with bodily fluid of a user; and
   determining, by the processor using the first analyte data, a first analyte level of the user at the sample time;
   determining, by the processor using second analyte data, a second analyte level of the user at the sample time, wherein the second analyte data includes historical analyte data preceding the sample time;
   comparing, by the processor, the first analyte level and the second analyte level resulting in a comparison value;
   determining, by the processor, the existence of a potential event if the comparison value exceeds a threshold value; **characterized by**:

   causing, by the processor, a request for confirmation of the potential event to be displayed to the user; and
   treating, by the processor, the first analyte data as more reliable if the potential event is confirmed by the user.

2. The method of claim 1, wherein the potential event is a meal, an insulin dose, or exercise.

3. The method of claim 1, further comprising:
   receiving, by the processor (307), a user tag of the event.

4. The method of claim 1, wherein the second analyte data includes user tagged information, optionally wherein the user tagged information includes meal information, insulin information, or exercise information.

5. The method of claim 1, further comprising:

   excluding, by the processor (307) if the potential event is confirmed by the user, the first analyte data from an artifact detection process or an artifact rejection process and/or
   subjecting, by the processor if the potential event is not confirmed by the user, the first analyte data to an artifact detection process or an artifact rejection process.

6. The method of claim 1, wherein determining, by the processor (307) using second analyte data, the second analyte level of the user at the sample time comprises estimating the second analyte level of the user at the sample time.

7. An analyte monitoring system (100), comprising:

a display (222, 310);

at least one processor (307); and

non-transitory memory on which is stored instructions that, when executed by the at least one processor, cause the at least one processor to:

determine, using first analyte data generated by an in vivo analyte sensor (110, 211) at a sample time, a first analyte level of the user at the sample time;

the system being **characterized in that** the instructions, when executed by the at least one processor, cause the at least one processor to:

determine, using second analyte data, a second analyte level of the user at the sample time, wherein the second analyte data includes historical analyte data preceding the sample time;

determine a comparison value from a comparison of the first analyte level and the second analyte level;

determine the existence of a potential event if the comparison value exceeds a threshold value;

cause a request for confirmation of the potential event to be displayed to the user; and

treat the first analyte data as more reliable if the potential event is confirmed by the user.

**8.** The system of claim 7, wherein the potential event is a meal, an insulin dose, or exercise.

**9.** The system of claim 7, wherein the instructions, when executed by the at least one processor (307), cause the at least one processor to process a user tag of the event.

**10.** The system of claim 7, wherein the second analyte data includes user tagged information, optionally wherein the user tagged information includes meal information, insulin information, or exercise information.

**11.** The system of claim 7, wherein the instructions, when executed by the at least one processor (307), cause the at least one processor to exclude, if the potential event is confirmed by the user, the first analyte data from an artifact detection process or an artifact rejection process and/or wherein the instructions, when executed by the at least one processor, cause the at least one processor to subject, if the potential event is not confirmed by the user, the first analyte data to an artifact detection process or an artifact rejection process.

**12.** The system of claim 7, wherein the instructions, when executed by the at least one processor (307), cause the at least one processor to estimate the second analyte level of the user at the sample time.

**Patentansprüche**

**1.** Ein computerimplementiertes Verfahren, das Folgendes beinhaltet:

Empfangen, durch einen Prozessor (307) eines Analytenüberwachungssystems (104, 220), von ersten Analytendaten, die von einem In-vivo-Analytensensor (110, 211) zum Zeitpunkt einer Probennahme erzeugt wurden, wobei der In-vivo-Analytensensor in Kontakt mit Körperflüssigkeit eines Benutzers steht; und

Bestimmen, durch den Prozessor unter Verwendung der ersten Analytendaten, einer ersten Analytenkonzentration des Benutzers zum Zeitpunkt der Probennahme;

Bestimmen, durch den Prozessor unter Verwendung von zweiten Analytendaten, einer zweiten Analytenkonzentration des Benutzers zum Zeitpunkt der Probennahme, wobei die zweiten Analytendaten historische Analytendaten, die vor dem Zeitpunkt der Probennahme liegen, umfassen;

Vergleichen, durch den Prozessor, der ersten Analytenkonzentration und der zweiten Analytenkonzentration, woraus sich ein Vergleichswert ergibt;

Bestimmen, durch den Prozessor, der Existenz eines potentiellen Ereignisses, wenn der Vergleichswert einen Schwellenwert überschreitet; **gekennzeichnet durch**:

Bewirken, durch den Prozessor, dass dem Benutzer eine Anforderung zum Bestätigen des potentiellen Ereignisses angezeigt wird; und

Behandeln, durch den Prozessor, der ersten Analytendaten als zuverlässiger, wenn das potentielle Ereignis von dem Benutzer bestätigt wird.

**2.** Verfahren nach Anspruch 1, wobei das potentielle Ereignis eine Mahlzeit, eine Insulindosis oder körperliche Betätigung ist.

3. Verfahren nach Anspruch 1, das ferner Folgendes beinhaltet:
Empfangen, durch den Prozessor (307), einer Benutzermarkierung des Ereignisses.

4. Verfahren nach Anspruch 1, wobei die zweiten Analytendaten vom Benutzer markierte Informationen umfassen, wobei die vom Benutzer markierten Informationen optional Mahlzeitinformationen, Insulininformationen oder Informationen zu körperlicher Betätigung umfassen.

5. Verfahren nach Anspruch 1, das ferner Folgendes beinhaltet:

Ausschließen, durch den Prozessor (307), falls das potentielle Ereignis von dem Benutzer bestätigt wird, der ersten Analytendaten aus einem Prozess zum Detektieren von Artefakten oder einem Prozess zum Ablehnen von Artefakten und/oder
Unterziehen, durch den Prozessor, falls das potentielle Ereignis nicht von dem Benutzer bestätigt wird, der ersten Analytendaten einem Prozess zum Detektieren von Artefakten oder einem Prozess zum Ablehnen von Artefakten.

6. Verfahren nach Anspruch 1, wobei das Bestimmen, durch den Prozessor (307) unter Verwendung von zweiten Analytendaten, der zweiten Analytenkonzentration des Benutzers zum Zeitpunkt der Probennahme das Schätzen der zweiten Analytenkonzentration des Benutzers zum Zeitpunkt der Probennahme beinhaltet.

7. Ein Analytenüberwachungssystem (100), das Folgendes beinhaltet:

eine Anzeige (222, 310);
mindestens einen Prozessor (307); und
nichtflüchtigen Speicher, in dem Anweisungen gespeichert sind, die, wenn sie von dem mindestens einen Prozessor ausgeführt werden, bewirken, dass der mindestens eine Prozessor Folgendes tut:
Bestimmen, unter Verwendung der ersten Analytendaten, die von einem In-vivo-Analytensensor (110, 211) zum Zeitpunkt einer Probennahme erzeugt wurden, einer ersten Analytenkonzentration des Benutzers zum Zeitpunkt der Probennahme;
wobei das System **dadurch gekennzeichnet ist, dass** die Anweisungen, wenn sie von dem mindestens einen Prozessor ausgeführt werden, bewirken, dass der mindestens eine Prozessor Folgendes tut:

Bestimmen, unter Verwendung von zweiten Analytendaten, einer zweiten Analytenkonzentration des Benutzers zum Zeitpunkt der Probennahme, wobei die zweiten Analytendaten historische Analytendaten, die vor dem Zeitpunkt der Probennahme liegen, umfassen;
Bestimmen eines Vergleichswerts aus einem Vergleich der ersten Analytenkonzentration und der zweiten Analytenkonzentration;
Bestimmen der Existenz eines potentiellen Ereignisses, wenn der Vergleichswert einen Schwellenwert überschreitet;
Bewirken, dass dem Benutzer eine Anforderung zum Bestätigen des potentiellen Ereignisses angezeigt wird; und
Behandeln der ersten Analytendaten als zuverlässiger, wenn das potentielle Ereignis von dem Benutzer bestätigt wird.

8. System nach Anspruch 7, wobei das potentielle Ereignis eine Mahlzeit, eine Insulindosis oder körperliche Betätigung ist.

9. System nach Anspruch 7, wobei die Anweisungen, wenn sie von dem mindestens einen Prozessor (307), ausgeführt werden, bewirken, dass der mindestens eine Prozessor eine Benutzermarkierung des Ereignisses verarbeitet.

10. System nach Anspruch 7, wobei die zweiten Analytendaten vom Benutzer markierte Informationen umfassen, wobei die vom Benutzer markierten Informationen optional Mahlzeitinformationen, Insulininformationen oder Informationen zu körperlicher Betätigung umfassen.

11. System nach Anspruch 7, wobei die Anweisungen, wenn sie von dem mindestens einen Prozessor (307) ausgeführt werden, bewirken, dass der mindestens eine Prozessor, falls das potentielle Ereignis von dem Benutzer bestätigt wird, die ersten Analytendaten aus einem Prozess zum Detektieren von Artefakten oder einem Prozess zum Ablehnen von Artefakten ausschließt, und/oder wobei die Anweisungen, wenn sie von dem mindestens einen Prozessor ausgeführt werden, bewirken, dass der mindestens eine Prozessor, falls das potentielle Ereignis nicht von dem Benutzer bestätigt wird, die ersten Analytendaten einem Prozess zum Detektieren von Artefakten oder einem Prozess zum Ablehnen von Artefakten unterzieht.

**12.** System nach Anspruch 7, wobei die Anweisungen, wenn sie von dem mindestens einen Prozessor (307) ausgeführt werden, bewirken, dass der mindestens eine Prozessor die zweite Analytenkonzentration des Benutzers zum Zeitpunkt der Probennahme schätzt.

**Revendications**

**1.** Procédé mis en œuvre par ordinateur comprenant :

la réception, par un processeur (307) d'un système de surveillance d'analyte (104, 220), de premières données d'analyte générées par un détecteur d'analyte in vivo (110, 211) à un premier temps d'échantillonnage, dans lequel le détecteur d'analyte in vivo est en contact avec un fluide corporel d'un utilisateur ; et
la détermination, par le processeur utilisant les premières données d'analyte, d'un premier niveau d'analyte de l'utilisateur au temps d'échantillonnage ;
la détermination, par le processeur utilisant des deuxièmes données d'analyte, d'un deuxième niveau d'analyte de l'utilisateur au temps d'échantillonnage, dans lequel les deuxièmes données d'analyte incluent des données d'analyte historiques précédant le temps d'échantillonnage ;
la comparaison, par le processeur, du premier niveau d'analyte et du deuxième niveau d'analyte ayant pour résultat une valeur de comparaison ;
la détermination, par le processeur, de l'existence d'un événement potentiel si la valeur de comparaison dépasse une valeur seuil ;
**caractérisé par** :

le fait d'amener, par le processeur, une requête de confirmation de l'événement potentiel à être affichée pour l'utilisateur ; et
le traitement, par le processeur, des premières données d'analyte comme étant plus fiables si l'événement potentiel est confirmé par l'utilisateur.

**2.** Procédé selon la revendication 1, dans lequel l'événement potentiel est un repas, une dose d'insuline, ou une activité physique.

**3.** Procédé selon la revendication 1, comprenant en outre :
la réception, par le processeur (307), d'une étiquette d'utilisateur de l'événement.

**4.** Procédé selon la revendication 1, dans lequel les deuxièmes données d'analyte incluent des informations étiquetées d'utilisateur, facultativement dans lequel les informations étiquetées d'utilisateur incluent des informations sur un repas, des informations sur de l'insuline, ou des informations sur une activité physique.

**5.** Procédé selon la revendication 1, comprenant en outre :

l'exclusion, par le processeur (307) si l'événement potentiel est confirmé par l'utilisateur, des premières données d'analyte d'un processus de détection d'artéfact ou d'un processus de rejet d'artéfact et/ou
la soumission, par le processeur si l'événement potentiel n'est pas confirmé par l'utilisateur, des premières données d'analyte à un processus de détection d'artéfact ou un processus de rejet d'artéfact.

**6.** Procédé selon la revendication 1, dans lequel la détermination, par le processeur (307) utilisant des deuxièmes données d'analyte, du deuxième niveau d'analyte de l'utilisateur au temps d'échantillonnage comprend l'estimation du deuxième niveau d'analyte de l'utilisateur au temps d'échantillonnage.

**7.** Système de surveillance d'analyte (100), comprenant :

un dispositif d'affichage (222, 310) ;
au moins un processeur (307) ; et
une mémoire non transitoire sur laquelle sont stockées des instructions qui, quand elles sont exécutées par l'au moins un processeur, amènent l'au moins un processeur à :

déterminer, en utilisant des premières données d'analyte générées par un détecteur d'analyte in vivo (110, 211) à un temps d'échantillonnage, un premier niveau d'analyte de l'utilisateur au temps d'échantillonnage ;

le système étant **caractérisé en ce que** les instructions, quand elles sont exécutées par l'au moins un processeur, amènent l'au moins un processeur à :

déterminer, en utilisant des deuxièmes données d'analyte, un deuxième niveau d'analyte de l'utilisateur au temps d'échantillonnage, dans lequel les deuxièmes données d'analyte incluent des données d'analyte historiques précédant le temps d'échantillonnage ;

déterminer une valeur de comparaison à partir d'une comparaison du premier niveau d'analyte et du deuxième niveau d'analyte ;

déterminer l'existence d'un événement potentiel si la valeur de comparaison dépasse une valeur seuil ;

amener une requête de confirmation de l'événement potentiel à être affichée pour l'utilisateur ; et

traiter les premières données d'analyte comme étant plus fiables si l'événement potentiel est confirmé par l'utilisateur.

8. Système selon la revendication 7, dans lequel l'événement potentiel est un repas, une dose d'insuline, ou une activité physique.

9. Système selon la revendication 7, dans lequel les instructions, quand elles sont exécutées par l'au moins un processeur (307), amènent l'au moins un processeur à traiter une étiquette d'utilisateur de l'événement.

10. Système selon la revendication 7, dans lequel les deuxièmes données d'analyte incluent des informations étiquetées, facultativement dans lequel les informations étiquetées d'utilisateur incluent des informations sur un repas, des informations sur de l'insuline, ou des informations sur une activité physique.

11. Système selon la revendication 7, dans lequel les instructions, quand elles sont exécutées par l'au moins un processeur (307), amènent l'au moins un processeur à exclure, si l'événement potentiel est confirmé par l'utilisateur, les premières données d'analyte d'un processus de détection d'artéfact ou d'un processus de rejet d'artéfact et/ou dans lequel les instructions, quand elles sont exécutées par l'au moins un processeur, amènent l'au moins un processeur à soumettre, si l'événement potentiel n'est pas confirmé par l'utilisateur, les premières données d'analyte à un processus de détection d'artéfact ou un processus de rejet d'artéfact.

12. Système selon la revendication 7, dans lequel les instructions, quand elles sont exécutées par l'au moins un processeur (307), amènent l'au moins un processeur à estimer le deuxième niveau d'analyte de l'utilisateur au temps d'échantillonnage.

FIG. 1

FIG. 2

FIG. 3

```
                    ┌─────────────┐
                    │   START     │
                    └─────────────┘
                           │
                           ▼
         ┌──────────────────────────────────┐
         │  Receive analyte data related to an │
         │          analyte level            │
         │               402                 │
         └──────────────────────────────────┘
                           │
                           ▼
         ┌──────────────────────────────────┐
         │  Determine an analyte level based upon │
         │     the received analyte data     │
         │               404                 │
         └──────────────────────────────────┘
                           │
                           ▼
         ┌──────────────────────────────────┐
         │  Determine a rate of change of the │
         │  analyte level using the received analyte │
         │    data and prior analyte data    │
         │               406                 │
         └──────────────────────────────────┘
                           │
                           ▼
         ┌──────────────────────────────────┐
         │  Categorize the analyte data related to │
         │  the analyte level into at least one bin │
         │    based upon the determined rate of │
         │      change of the analyte level  │
         │               408                 │
         └──────────────────────────────────┘
                           │
                           ▼
         ┌──────────────────────────────────┐
         │  Determine a recommended insulin dose │
         │  based upon the determined analyte level │
         │               410                 │
         └──────────────────────────────────┘
                           │
                           ▼
         ┌──────────────────────────────────┐
         │  Modify the recommended insulin dose │
         │      based at least in part on the │
         │  categorization of the analyte data into │
         │        the at least one bin       │
         │               412                 │
         └──────────────────────────────────┘
                           │
                           ▼
         ┌──────────────────────────────────┐
         │  Present the modified recommended │
         │          insulin dose             │
         │               414                 │
         └──────────────────────────────────┘
                           │
                           ▼
                    ┌─────────────┐
                    │    END      │
                    └─────────────┘
```

FIG. 4

FIG. 5

START

Receive analyte data related to
an analyte level
602

Determine an analyte level
based upon the received analyte
data
604

Determine a rate of change of
the analyte level using the
received analyte data and prior
analyte data
606

Determine a recommended
insulin dose based upon the
determined analyte level
608

Modify the recommended insulin
dose based upon at least one of
a lag between an interstitial fluid
analyte level and blood analyte
level and insulin sensitivity
610

Present the modified
recommended insulin dose
612

END

FIG. 6

START

Receive analyte data related to
an analyte level
702

Determine an analyte level
based upon the received analyte
data
704

Determine a rate of change of
the analyte level using the
received analyte data and prior
analyte data.
706

Determine a recommended
insulin dose based upon the
determined analyte level
708

Determine an analyte excursion
for a predetermined future time
using at least one of the
determined rate of change and a
prior rate of change
710

Modify the recommended insulin
dose based upon the determined
analyte extrusion for the
predetermined future time
712

Present the modified
recommended insulin dose
714

END

**FIG. 7**

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2009143725 A
- US 2010057042 A
- US 20060193375 A
- US 5262035 A
- US 5264104 A
- US 5262305 A
- US 5320715 A
- US 5593852 A
- US 6175752 B

- US 6650471 B
- US 6746582 B
- US 6284478 B
- US 7299082 B
- US 10745878 B
- US 11060365 B
- US 12698124 B
- US 7630748 B

### Non-patent literature cited in the description

- Physiological Evaluation of Factors Controlling Glucose Tolerance in Man, Measurement of Insulin Sensitivity and Beta-cell Glucose Sensitivity From the Response to Intravenous Glucose. *J. Clin. Invest., The American Society for Clinical Investigation, Inc.,* December 1981, vol. 68, 1456-1467

- **UMENO et al.** Robust Speed Control of DC Servomotors using Modern Two-Degrees- of-Freedom Controller Design. *IEEE Trans, on Industrial Electronics,* 1991, vol. 38 (5), 363-368
- **WINTERS.** Forecasting Sales by Exponentially Weighted Moving Averages. *MANAGEMENT SCIENCE,* 01 April 1960, vol. 6, 324-342